(19) Europäisches Patentamt
European Patent Office
Office européen des brevets

(11) Publication number: **0 500 264 B1**

# (12) EUROPEAN PATENT SPECIFICATION

(45) Date of publication of patent specification: **15.11.95**

(51) Int. Cl.6: **A01N 43/72**, A01N 63/04, //(C12P21/04,C12R1:645)

(21) Application number: **92301186.0**

(22) Date of filing: **13.02.92**

## (54) Agricultural and horticultural fungicides.

(30) Priority: **19.02.91 JP 47664/91**
**15.11.91 JP 327996/91**

(43) Date of publication of application:
**26.08.92 Bulletin 92/35**

(45) Publication of the grant of the patent:
**15.11.95 Bulletin 95/46**

(84) Designated Contracting States:
**DE ES FR GB IT**

(56) References cited:
**EP-A- 0 352 092**
**EP-A- 0 443 719**

**PATENT ABSTRACTS OF JAPAN, unexamined applications, section C, vol. 15, no.177, May 7, 1991, THE PATENT OFFICE JAPANESE GOVERNMENT page 44 C 829**

(73) Proprietor: **TAKARA SHUZO CO. LTD.**
**609 Takenakacho**
**Fushimi-ku**
**Kyoto (JP)**

(72) Inventor: **Takesako, Kazutoh**
**10-5-1053, Nishioji-cho**
**Kusatsu-shi,**
**Shiga-ken (JP)**
Inventor: **Kato, Ikunoshin**
**1-1-150, Nanryo-cho**
**Uji-shi,**
**Kyoto-fu (JP)**
Inventor: **Endo, Masahiro**
**2-1-15-201, Seta**
**Otsu-shi,**
**Shiga-ken (JP)**

(74) Representative: **Marlow, Nicholas Simon et al**
**Reddie & Grose**
**16, Theobalds Road**
**London WC1X 8PL (GB)**

EP 0 500 264 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to an agricultural and horticultural fungicide comprising as an active ingredient an R106 compound which has low toxicity and a high antifungal activity.

DESCRIPTION OF THE PRIOR ART

As agricultural and horticultural fungicides, many synthetic compounds and antibiotics are heretofore known. For example, validamycin, blasticidin S, polyoxin, kasugamycin and the like have been used as antibiotics for the purpose of controlling plant diseases caused by fungi. However, these fungicides are not necessarily satisfactory in effectiveness, development of resistant fungi, etc.

It has been desired to provide an agricultural and horticultural fungicide which does not adversely affect crops and protects fruit trees and crops such as vegetables from plant diseases mainly caused by fungi.

The present invention relates the use of R106 compounds represented by the following general formula (I):

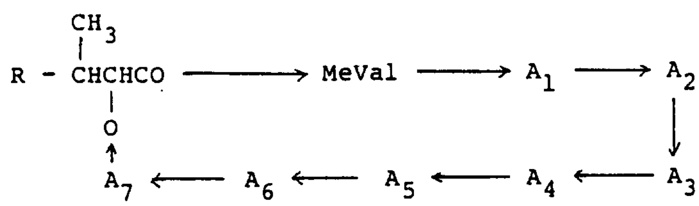

(I)

wherein:

R is methyl or ethyl;

$A_1$ is Phe, o-FPhe, m-FPhe or Tyr;

$A_2$ is MePhe, $\beta$-HOMePhe, Phe, o-FMePhe, m-FMePhe, MeTyr, Sar, MeSer, $\beta$-oxoMePhe or $\beta$-$R_2$OMePhe (wherein $R_2$ is a lower acyl group having 1 to 4 carbon atoms);

$A_3$ is Pro, 4Hyp or SPro;

$A_4$ is allo-Ile, Val, Leu or Nle;

$A_5$ is MeVal or Val;

$A_5$ is Leu or Nva;

$A_7$ is $\beta$-HOMeVal, $\gamma$-HOMeVal, MeVal, Val, N,$\beta$-MeAsp, $\beta$-HOMePhe, MePhe, MeDH$_{2,3}$Val, MeDH$_{3,4}$Val or Sar;

as an active ingredient in an agricultural and/or horticultural fungicide.

Abbreviations for amino acids used in the general formula described above are given below.

Phe: phenylalanine
o-FPhe: o-fluorophenylalanine
m-FPhe: m-fluorophenylalanine
MePhe: N-methylphenylalanine
$\beta$-HOMePhe: $\beta$-hydroxy-N-methylphenylalanine
$\beta$-$R_2$OMePhe: $\beta$-acyloxy-N-methylphenylalanine
$\beta$-AcOMePhe: $\beta$-acetoxy-N-methylphenylalanine
$\beta$-oxoMePhe: $\beta$-oxo-N-methylphenylalanine
o-FMePhe: o-fluoro-N-methylphenylalanine
m-FMePhe: m-fluoro-N-methylphenylalanine
Tyr: tyrosine
MeTyr: N-methyltyrosine
Val: valine
MeVal: N-methylvaline
Nva: norvaline
$\beta$-HOMeVal: $\beta$-hydroxy-N-methylvaline
$\gamma$-HOMeVal: $\gamma$-hydroxy-N-methylvaline
MeDH$_{2,3}$Val: N-methyl-2,3-didehydrovaline
MeDH$_{3,4}$Val: N-methyl-3,4-didehydrovaline
allo-Ile: alloisoleucine

Leu: leucine
Nle: norleucine
N,β-MeAsp: N,β-dimethylaspartic acid
Pro: proline
4Hyp: 4-hydroxyproline
SPro: thioproline
Sar: sarcosine
MeSer: N-methylserine

The present inventors have made investigations on the R106 compounds that were already disclosed as novel antibiotics having low toxicity in U.S. Patent Appln. 07/379629, Japan Laid-Open Patent Appln. 3-44398, and U.S. Patent Appln. 07/643948 and as a result, have found that the R106 compounds show an extremely excellent control activity against plant diseases caused by various fungi and are useful as agricultural and horticultural fungicides. The present invention has thus been accomplished. In the present invention, at least one compound selected from the R106 compounds is used and a mixture of two or more of the R106 compounds may also be used.

## DETAILED DESCRIPTION OF THE PREFERRED EMBODIMENTS

The R106 compounds used in the present invention can be prepared by the process described in the specifications described above using as raw materials the culture broth of Aureobasidium pullulans No. R106 [FERM BP-1938] or compounds obtained by the culture broth.

Representative examples of the R106 compounds shown by the general formula (I) described above which can be used in the present invention are given in Table 1 below.

Table 1

| Compound | $R_1$ | $A_1$ | $A_2$ | $A_3$ | $A_4$ | $A_5$ | $A_6$ | $A_7$ |
|---|---|---|---|---|---|---|---|---|
| 1 | $C_2H_5$ | Phe | MePhe | Pro | allo-Ile | MeVal | Leu | β-HOMeVal |
| 2 | $CH_3$ | Phe | MePhe | Pro | allo-Ile | MeVal | Leu | β-HOMeVal |
| 3 | $C_2H_5$ | Phe | MePhe | Pro | Val | MeVal | Leu | β-HOMeVal |
| 4 | $C_2H_5$ | Phe | MePhe | Pro | allo-Ile | MeVal | Leu | γ-HOMeVal |
| 5 | $C_2H_5$ | Phe | β-HOMePhe | Pro | allo-Ile | MeVal | Leu | β-HOMeVal |
| 6 | $C_2H_5$ | Phe | MePhe | Pro | allo-Ile | Val | Leu | β-HOMeVal |
| 7 | $C_2H_5$ | Phe | MePhe | Pro | allo-Ile | MeVal | Leu | MeVal |
| 8 | $C_2H_5$ | Phe | MePhe | Pro | allo-Ile | MeVal | Leu | Val |
| 9 | $C_2H_5$ | Phe | MePhe | Pro | Leu | MeVal | Leu | β-HOMeVal |
| 10 | $C_2H_5$ | Phe | MePhe | Pro | allo-Ile | MeVal | Leu | N,β-MeAsp |
| 11 | $CH_3$ | Phe | MePhe | Pro | allo-Ile | MeVal | Leu | MeVal |
| 12 | $C_2H_5$ | Phe | MePhe | Pro | Val | MeVal | Leu | MeVal |
| 13 | $C_2H_5$ | Phe | Phe | Pro | allo-Ile | MeVal | Leu | MeVal |
| 14 | $C_2H_5$ | Phe | MePhe | Pro | allo-Ile | MeVal | Leu | $MeDH_{3,4}Val$ |
| 15 | $C_2H_5$ | Phe | MePhe | Pro | allo-Ile | MeVal | Leu | β-HOMePhe |
| 16 | $C_2H_5$ | Phe | MePhe | Pro | allo-Ile | Val | Leu | MeVal |
| 17 | $C_2H_5$ | Phe | MePhe | Pro | allo-Ile | MeVal | Leu | MePhe |

| | | | | | | | | |
|---|---|---|---|---|---|---|---|---|
| 18 | $C_2H_5$ | Phe | MePhe | Pro | allo-Ile | MeVal | Leu | $MeDH_{2,3}Val$ |
| 19 | $C_2H_5$ | o-FPhe | o-FMePhe | Pro | allo-Ile | MeVal | Leu | ß-HOMeVal |
| 20 | $C_2H_5$ | m-FPhe | m-FMePhe | Pro | allo-Ile | MeVal | Leu | ß-HOMeVal |
| 21 | $C_2H_5$ | Tyr | MeTyr | Pro | allo-Ile | MeVal | Leu | ß-HOMeVal |
| 22 | $C_2H_5$ | Phe | MePhe | 4Hyp | allo-Ile | MeVal | Leu | ß-HOMeVal |
| 23 | $C_2H_5$ | Phe | MePhe | SPro | allo-Ile | MeVal | Leu | ß-HOMeVal |
| 24 | $C_2H_5$ | Phe | MePhe | Pro | Nle | MeVal | Leu | ß-HOMeVal |
| 25 | $C_2H_5$ | Phe | MePhe | Pro | allo-Ile | MeVal | Nva | ß-HOMeVal |
| 26 | $C_2H_5$ | Phe | Sar | Pro | allo-Ile | MeVal | Leu | ß-HOMeVal |
| 27 | $C_2H_5$ | Phe | MeSer | Pro | allo-Ile | MeVal | Leu | ß-HOMeVal |
| 28 | $C_2H_5$ | Phe | MePhe | Pro | allo-Ile | MeVal | Leu | Sar |
| 29 | $C_2H_5$ | Phe | Sar | Pro | allo-Ile | MeVal | Leu | Sar |
| 30 | $C_2H_5$ | Phe | ß-oxoMePhe | Pro | allo-Ile | MeVal | Leu | ß-HOMeVal |
| 31 | $C_2H_5$ | Phe | ß-AcOMePhe | Pro | allo-Ile | MeVal | Leu | ß-HOMeVal |

As plant diseases against which the compounds used in the present invention exhibit an excellent effect, there are, for example, gray mold disease by Botrytis cinera in a cucumber, a tomato or a strawberry; powdery mildew disease by Erysiphe graminis in a barley, black soot disease by Alternaria porri in a cabbage, black spot disease by Alternaria kikuchiana in a pear, cork spot disease by Alternaria mali in an apple, etc.

Where the compounds described above are used in the present invention as the active ingredient, one or two or more may be used simultaneously. The compounds per se may be applied directly to plants but generally used by blending them with a solid carrier, a liquid carrier, a surface active agent, an extender and other adjuvants for preparation. That is, as in ordinary agricultural chemicals, the compounds may be prepared into an emulsifiable concentrate, a water dispersible powder or wettable powder, a granule, a powder, liquid, etc., which are provided for use.

As these carriers, mention may be made of clay, kaolin, bentonite, acid clay, diatomaceous earth or calcium carbonate; solid carriers such as nitrocellulose, starch, or gum arabic; liquid carriers such as water, methanol, ethanol, acetone, dimethylformamide, or ethylene glycol; In addition, adjuvants which are conventionally used for preparations may also be appropriately blended; examples of these adjuvants include higher alcohol sulfuric acid esters, polyoxyethylene, alkylaryl ethers, alkylaryl polyethylene glycol ethers, alkylaryl sorbitan monolaurates, alkylaryl sulfonates, alkylaryl sulfonates, quaternary ammonium salts, and polyalkylene oxide.

The active ingredient may be appropriately blended in an amount of 10 to 90% for the emulsifiable concentrate and wettable powder and in an amount of 0.1 to 10% for the powder and oil spray. These concentrations may be appropriately varied depending upon the purpose of use.

The resulting fungicide may also be blended with other fungicides, herbicides, insecticides, fertilizers, soil conditioners, etc.

The present invention is described with reference to the examples below.

Example 1

The minimum inhibition concentration (MIC) of various compounds used in the present invention against various plant pathogenic fungi was determined.

MIC was determined by agar plate dilution method using potato dextrose agar medium (manufactured by Nissui Pharmaceutical Co., Ltd.). Incubation was carried out at 27 °C for 4 days.

The results are shown in Table 2.

Table 2

| Fungi assayed | IFO No. | MIC (μg/ml) Compound No. 1 | 2 | 3 | 4 | 5 | 6 | 7 |
|---|---|---|---|---|---|---|---|---|
| Pellicularia filamentosa | 5254 | 12.5 | >25 | 25 | 6.25 | 25 | >25 | >25 |
| Pyricularia oryzae | 5279 | 0.39 | 1.56 | 1.56 | >25 | >25 | >25 | >25 |
| Botrytis cinera | 31386 | < 0.10 | 0.20 | 0.20 | 0.78 | 0.39 | 0.78 | 25 |
| Alternaria porri | 9762 | 0.20 | 0.78 | 1.56 | 6.25 | 0.78 | 6.25 | 25 |

| Fungi assayed | IFO No. | MIC (μg/ml) Compound No. 8 | 9 | 10 | 11 | 12 | 13 | 14 |
|---|---|---|---|---|---|---|---|---|
| Pellicularia filamentosa | 5254 | >100 | 12.5 | >100 | >100 | >100 | >100 | >100 |
| Pyricularia oryzae | 5279 | >100 | 0.39 | 50 | >100 | >100 | >100 | 100 |
| Botrytis cinera | 31386 | 25 | 0.10 | 6.25 | 25 | 50 | 50 | 25 |
| Alternaria porri | 9762 | 50 | 0.20 | 12.5 | 50 | >100 | >100 | >100 |

| Fungi assayed | IFO No. | MIC (µg/ml) Compound No. 15 | 16 | 17 | 18 | 19 | 20 | 21 |
|---|---|---|---|---|---|---|---|---|
| Pellicularia filamentosa | 5254 | >100 | >100 | >100 | >100 | 25 | 25 | 25 |
| Pyricularia oryzae | 5279 | >100 | >100 | >100 | 100 | 100 | 1.56 | 3.12 |
| Botrytis cinera | 31386 | 50 | 50 | 50 | 50 | 0.20 | 0.20 | 0.20 |
| Alternaria porri | 9762 | >100 | 100 | >100 | >100 | 1.56 | 1.56 | 1.56 |

| Fungi assayed | IFO No. | MIC (µg/ml) Compound No. 22 | 23 | 24 | 25 | 26 | 27 | 28 |
|---|---|---|---|---|---|---|---|---|
| Pellicularia filamentosa | 5254 | 12.5 | 12.5 | 50 | 25 | 25 | >100 | >100 |
| Pyricularia oryzae | 5279 | 1.56 | 3.12 | 3.12 | 3.12 | 6.25 | 50 | >100 |
| Botrytis cinera | 31386 | 0.10 | 0.10 | 0.20 | 0.10 | 0.10 | 0.25 | 50 |
| Alternaria porri | 9762 | 0.78 | 0.78 | 3.12 | 1.56 | 0.78 | 25 | >100 |

| Fungi assayed | IFO No. | MIC (µg/ml) Compound No. 29 | 30 | 31 |
|---|---|---|---|---|
| Pellicularia filamentosa | 5254 | >100 | 25 | 100 |
| Pyricularia oryzae | 5279 | >100 | 1.56 | 50 |
| Botrytis cinera | 31386 | 50 | 0.10 | 0.39 |
| Alternaria porri | 9762 | >100 | 3.12 | 1.56 |

As is evident from Table 2, the R106 compounds showed an antifungal activity against various plant pathogenic fungi. In particular, the compounds showed an excellent activity against Botrytis cinera which is a pathogenic fungi of gray mold in cucumbers, etc.

Example 2

The control activity of Compound No. 1 used in the present invention on various plant diseases was examined using young plants grown in pots.

The test suspension was prepared by dissolving a test compound in acetone, adding 9-fold volume of water containing 100 ppm of extender (Hyten) to the solution to dilute the solution and subjecting the dilution to ultrasonic treatment.

The test on control activity was performed by sufficiently applying the test suspension (containing 100 ppm of the active ingredient) to leaves and stems using an automatic applicator, air-drying and then inoculating the spore suspension of pathogenic fungi in tests on most diseases. As an exception, an agar

piece containing fungi was used as the inoculum in cabbage black soot. The inoculated plant was allowed to stand for 5 to 10 days in a hothouse for disease-emergence kept at the optimum temperature suitable for various diseases.

The degree of controlling plant diseases was judged based on the following evaluation.

5: 100%
4: 99-90%
3: 89-70%
2: 69-40%
1: less than 40%

The results obtained are shown in Table 3.

Table 3

| Pathogenic Fungi | Barley powdery mildew 1) | Wheat leaf rust 2) | Cucumber gray mold 3) | Cabbage black soot 4) |
|---|---|---|---|---|
| Evaluation | 4 | 3 | 5 | 5 |

Pathogenic fungi
1) Erysiphe graminis
2) Puccinia recondita
3) Botrytis cinera
4) Alternaria porri

As is clearly noted in Table 3, Compound No. 1 showed the control activity against various plant diseases.

Example 3

The control activity of Compound No. 1 used in the present invention on cucumber gray mold fungi was examined.

The test suspension of Compound No. 1 prepared in various concentrations in a manner similar to Example 2 was sprayed on leaves and stems of cucumbers grown in pots. Twenty four hours after, the agar pieces on which pathogenic fungi were grown were put on the leaves and allowed to stand in a wet house at 20 °C. Four days after inoculation, the diameter of a disease spot on the cucumber leaves was measured. The control value was determined by comparing with the intact group (diameter of a disease spot: 24.7 mm). The results obtained are shown in Table 4.

Table 4

| Compound | Concentration (ppm) | Control Value (%) |
|---|---|---|
| 1 | 100 | 100 |
| | 25 | 100 |
| | 6.3 | 62 |

Example 4 (wettable powder)

By blending 10 parts of Compound No. 1 used in the present invention, 5 parts of sodium lauryl sulfate, 2 parts of sodium dinaphthylmethane-disulfonate-formalin condensation product and 83 parts of clay and grinding the blend into powders, 100 parts of wettable powders are obtained.

Example 5 (emulsifiable concentrate)

By blending 8 parts of Compound No. 1 used in the present invention, 10 parts of ethylene glycol, 20 parts of dimethylformamide, 10 parts of alkyl dimethylbenzyl ammonium chloride and 52 parts of methanol and dissolving the blend, 100 parts of emulsifiable concentrate are obtained.

Example 6 (powder)

By blending 0.2 part of Compound No. 1 used in the present invention, 0.5 part of calcium steareate, 50 parts of talc and 49.3 parts of clay and grinding the blend into powders, 100 parts of wettable powders are obtained.

Example 7 (granule)

By blending 10 parts of Compound No. 1 used in the present invention, 15 parts of starch, 72 parts of bentonite and 3 parts of sodium salt of lauryl alcohol sulfuric acid ester and grinding the blend into powders, 100 parts of granules are obtained.

As stated above, the agricultural and horticultural fungicides comprising the R106 compounds have an antifungal activity against various pathogenic fungi.

## Claims

**1.** The use of R106 compound represented by the following general formula (I):

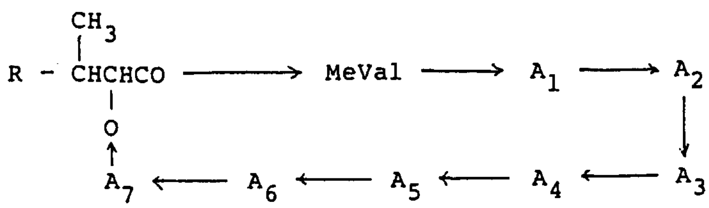

(I)

wherein:
R is methyl or ethyl;
A, is Phe, o-FPhe, m-FPhe or Tyr;
$A_2$ is MePHe, $\beta$-HOMePhe, Phe, o-FMePhe, m-FMePhe, MeTyr, Sar, MeSer, $\beta$-oxoMePhe or $\beta$-$R_2$OMePhe (wherein $R_2$ is a lower acyl group having 1 to 4 carbon atoms);
$A_3$ is Pro, 4Hyp or SPro;
$A_4$ is allo-Ile, Val, Leu or Nle;
$A_5$ is MeVal or Val;
$A_5$ is Leu or Nva;
$A_7$ is $\beta$-HOMeVal, $\gamma$-HOMeVal, MeVal, Val, N,$\beta$-MeAsp, $\beta$-HOMePhe, MePhe, MeD$H_{2,3}$Val, MeD$H_{3,4}$Val or Sar;
as an active ingredient in an agricultural and/or horticultural fungicide.

**2.** Method of preparing an agricultural and/or horticultural fungicide comprising the step of adding as an active ingredient RIO6 compound represented by the following general formula (I):

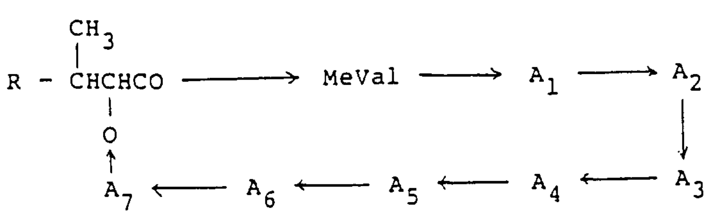

(I)

wherein:
R is methyl or ethyl;
$A_1$ is Phe, o-FPhe, m-FPhe or Tyr;
$A_2$ is MePHe, $\beta$-HOMePhe, Phe, o-FMePhe, m-FMePhe, MeTyr, Sar, MeSer, $\beta$-oxoMePhe or $\beta$-

$R_2$OMePhe (wherein $R_2$ is a lower acyl group having 1 to 4 carbon atoms);
$A_3$ is Pro, 4Hyp or SPro;
$A_4$ is allo-Ile, Val, Leu or Nle;
$A_5$ is MeVal or Val;
$A_5$ is Leu or Nva;
$A_7$ is $\beta$-HOMeVal, $\gamma$-HOMeVal, MeVal, Val, N,$\beta$-MeAsp, $\beta$-HOMePhe, MePhe, MeDH$_{2,3}$Val, MeDH$_{3,4}$Val or Sar;
to another agricultural and/or horticultural fungicide, herbicide, insecticide, fertilizer and/or soil conditioner.

**3.** An agricultural and/or horticultural fungicide composition comprising:
(i) as an active ingredient RIO6 compound represented by the following general formula (I):

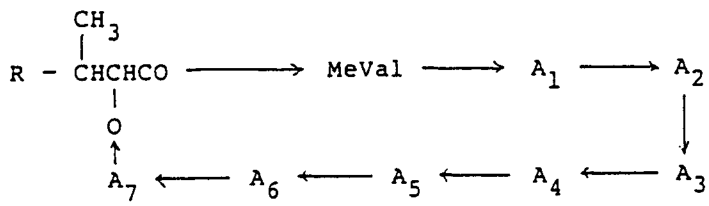
(I)

wherein:
R is methyl or ethyl;
$A_1$ is Phe, o-FPhe, m-FPhe or Tyr;
$A_2$ is MePHe, $\beta$-HOMePhe, Phe, o-FMePhe, m-FMePhe, MeTyr, Sar, MeSer, $\beta$-oxoMePhe or $\beta$-$R_2$OMePhe (wherein $R_2$ is a lower acyl group having 1 to 4 carbon atoms);
$A_3$ is Pro, 4Hyp or SPro;
$A_4$ is allo-Ile, Val, Leu or Nle;
$A_5$ is MeVal or Val;
$A_5$ is Leu or Nva;
$A_7$ is $\beta$-HOMeVal, $\gamma$-HOMeVal, MeVal, Val, N,$\beta$-MeAsp, $\beta$-HOMePhe, MePhe, MeDH$_{2,3}$Val, MeDH$_{3,4}$Val or Sar;
(ii) another agricultural and/or horticultural fungicide, herbicide, insecticide, fertilizer and/or soil conditioner.

### Patentansprüche

**1.** Verwendung der R106 Verbindung, dargestellt durch die folgende allgemeine Formel (I):

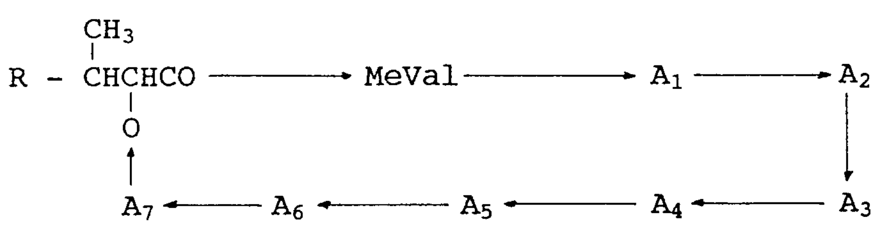
(I)

worin:
R Methyl oder Ethyl ist;
$A_1$ Phe, o-FPhe, m-FPhe oder Tyr ist;
$A_2$ MePhe, $\beta$-HOMePhe, Phe, o-FMePhe, m-FMePhe, MeTyr, Sar, MeSer, $\beta$-oxoMePhe oder $\beta$-$R_2$OMePhe ist (worin $R_2$ eine niedrige Acylgruppe mit 1 bis 4 Kohlenstoffatomen ist);
$A_3$ Pro, 4Hyp oder SPro ist;
$A_4$ allo-Ile, Val, Leu oder Nle ist;

$A_5$ MeVal oder Val ist;
$A_5$ Leu oder Nva ist;
$A_7$ $\beta$-HOMeVal, $\gamma$-HOMeVal, MeVal, Val, N,$\beta$-MeAsp,
$\beta$-HOMePhe, MePhe, MeDH$_{2,3}$Val, MeDH$_{3,4}$Val oder Sar ist; als ein wirksamer Bestandteil in einem Fungizid für den Acker- und/oder Gartenbau.

**2.** Verfahren zur Herstellung eines Fungizides für den Acker- und/oder Gartenbau umfassend den Schritt des Zusatzes der R106 Verbindung als wirksamen Bestandteil, dargestellt durch die folgende allgemeine Formel (I):

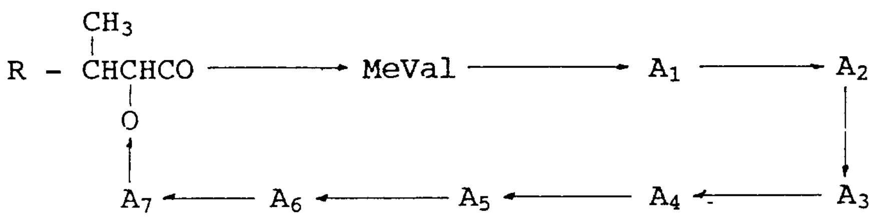

(I)

worin:
R Methyl oder Ethyl ist;
$A_1$ Phe, o-FPhe, m-FPhe oder Tyr ist;
$A_2$ MePhe, $\beta$-HOMePhe, Phe, o-FMePhe, m-FMePhe, MeTyr, Sar, MeSer, $\beta$-oxoMePhe oder $\beta$-$R_2$OMePhe ist (worin $R_2$ eine niedrige Acylgruppe mit 1 bis 4 Kohlenstoffatomen ist);
$A_3$ Pro, 4Hyp oder SPro ist;
$A_4$ allo-Ile, Val, Leu oder Nle ist;
$A_5$ MeVal oder Val ist;
$A_5$ Leu oder Nva ist;
$A_7$ $\beta$-HOMeVal, $\gamma$-HOMeVal, MeVal, Val, N,$\beta$-MeAsp,
$\beta$-HOMePhe, MePhe, MeDH$_{2,3}$Val, MeDH$_{3,4}$Val oder Sar ist; zu einem anderen Fungizid für den Acker- und/oder Gartenbau, Herbizid, Insektizid, Dünger und/oder Bodenkonditionierer.

**3.** Fungizidzusammensetzung für den Acker- und/oder Gartenbau umfassend:
(i) als wirksamen Bestandteil die R106 Verbindung, dargestellt durch die folgende allgemeine Formel (I):

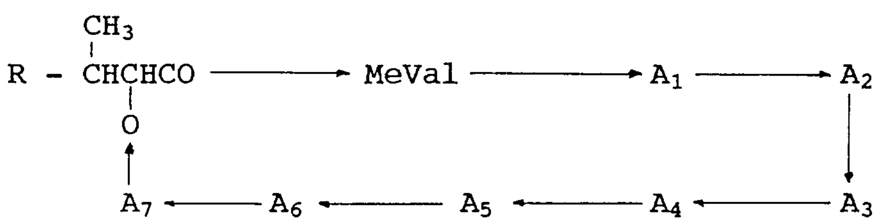

(I)

worin:
R Methyl oder Ethyl ist;
$A_1$ Phe, o-FPhe, m-FPhe oder Tyr ist;
$A_2$ Mephe, $\beta$-HOMePhe, Phe, o-FMePhe, m-FMePhe, MeTyr, Sar, MeSer, $\beta$-oxoMePhe oder $\beta$-$R_2$OMePhe ist (worin $R_2$ eine niedrige Acylgruppe mit 1 bis 4 Kohlenstoffatomen ist);
$A_3$ Pro, 4Hyp oder SPro ist;
$A_4$ allo-Ile, Val, Leu oder Nle ist;
$A_5$ MeVal oder Val ist;
$A_5$ Leu oder Nva ist;
$A_7$ $\beta$-HOMeVal, $\gamma$-HOMeVal, MeVal, Val, N,$\beta$-MeAsp,
$\beta$-HOMePhe, MePhe, MeDH$_{2,3}$Val, MeDH$_{3,4}$Val oder Sar ist;
(ii) ein anderes Fungizid für den Acker- und/oder Gartenbau, Herbizid, Insektizid, Dünger und/oder Bodenkonditionierer.

## Revendications

**1.** Utilisation du composé R106 représenté par la formule générale (I) suivante :

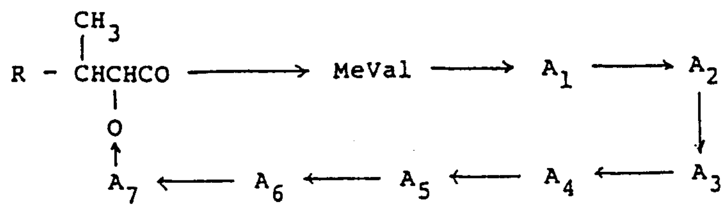

(I)

dans laquelle :

R représente un groupe méthyle ou éthyle ;

$A_1$ représente un groupe Phe, o-FPhe, m-FPhe ou Tyr ;

$A_2$ représente un groupe MePHe, $\beta$-HOMePhe, Phe, o-FMePhe, m-FMePhe, MeTyr, Sar, MeSer, $\beta$-oxoMePhe ou $\beta$-$R_2$OMePhe (dans lequel $R_2$ représente un groupe acyle inférieur ayant 1 à 4 atomes de carbone) ;

$A_3$ représente un groupe Pro, 4Hyp ou SPro ;

$A_4$ représente un groupe allo-Ile, Val, Leu ou Nle ;

$A_5$ représente un groupe MeVal ou Val ;

$A_5$ représente un groupe Leu ou Nva ;

$A_7$ représente un groupe $\beta$-HOMeVal, $\gamma$-HOMeVal, MeVal, Val, N,$\beta$-MeAsp, $\beta$-HOMePhe, MePhe, $MeDH_{2,3}$Val, $MeDH_{3,4}$Val ou Sar ;

comme ingrédient actif dans un fongicide destiné à être utilisé en agriculture et/ou en horticulture.

**2.** Procédé de préparation d'un fongicide destiné à être utilisé en agriculture et/ou en horticulture, comprenant l'étape d'addition d'un ingrédient actif consistant en le composé R106 représenté par la formule générale (I) suivante :

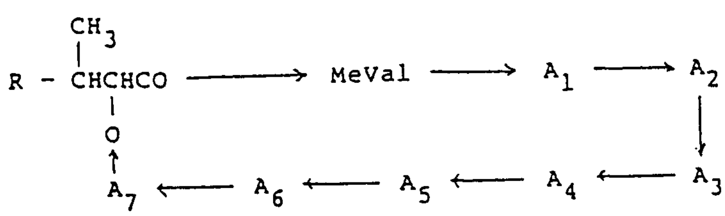

(I)

dans laquelle :

R représente un groupe méthyle ou éthyle ;

$A_1$ représente un groupe Phe, o-FPhe, m-FPhe ou Tyr ;

$A_2$ représente un groupe MePHe, $\beta$-HOMePhe, Phe, o-FMePhe, m-FMePhe, MeTyr, Sar, MeSer, $\beta$-oxoMePhe ou $\beta$-$R_2$OMePhe (dans lequel $R_2$ représente un groupe acyle inférieur ayant 1 à 4 atomes de carbone) ;

$A_3$ représente un groupe Pro, 4Hyp ou SPro ;

$A_4$ représente un groupe allo-Ile, Val, Leu ou Nle ;

$A_5$ représente un groupe MeVal ou Val ;

$A_5$ représente un groupe Leu ou Nva ;

$A_7$ représente un groupe $\beta$-HOMeVal, $\gamma$-HOMeVal, MeVal, Val, N,$\beta$-MeAsp, $\beta$-HOMePhe, MePhe, $MeDH_{2,3}$Val, $MeDH_{3,4}$Val ou Sar ;

à un autre fongicide destiné à être utilisé en agriculture et/ou en horticulture, un herbicide, un insecticide, un engrais et/ou un agent d'amendement du sol.

**3.** Composition de fongicide destinée à être utilisée en agriculture et/ou en horticulture, comprenant :

(i) comme ingrédient actif, un composé R106 représenté par la formule générale (I) suivante :

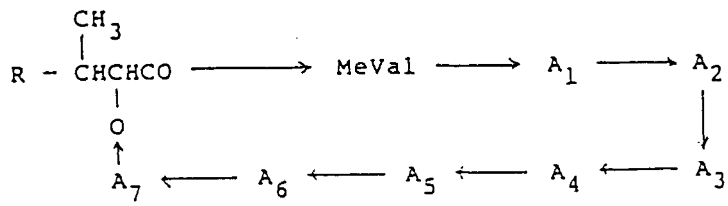

(I)

dans laquelle :

R représente un groupe méthyle ou éthyle ;

$A_1$ représente un groupe Phe, o-FPhe, m-FPhe ou Tyr ;

$A_2$ représente un groupe MePHe, $\beta$-HOMePhe, Phe, o-FMePhe, m-FMePhe, MeTyr, Sar, MeSer, $\beta$-oxoMePhe ou $\beta$-$R_2$OMePhe (dans lequel $R_2$ représente un groupe acyle inférieur ayant 1 à 4 atomes de carbone) ;

$A_3$ représente un groupe Pro, 4Hyp ou SPro ;

$A_4$ représente un groupe allo-Ile, Val, Leu ou Nle ;

$A_5$ représente un groupe MeVal ou Val ;

$A_5$ représente un groupe Leu ou Nva ;

$A_7$ représente un groupe $\beta$-HOMeVal, $\gamma$-HOMeVal, MeVal, Val, N,$\beta$-MeAsp, $\beta$-HOMePhe, MePhe, $MeDH_{2,3}$Val, $MeDH_{3,4}$Val ou Sar ;

(ii) un autre fongicide destiné à être utilisé en agriculture et/ou en horticulture, un herbicide, un insecticide, un engrais et/ou un agent d'amendement du sol.